# EUROPÄISCHE PATENTSCHRIFT

(11) **EP 2 194 940 B1**
(45) Veröffentlichungstag und Bekanntmachung des Hinweises auf die Patenterteilung: **21.09.2011**
(21) Anmeldenummer: 08758435.5
(22) Anmeldetag: 09.05.2008
(51) Int. Cl.: A61F 7/02

(54) **Temperiereinrichtung zum Temperieren von Hautpartien des menschlichen Körpers**
Temperature control device for controlling the temperature of areas of the skin of the human body
Dispositif de régulation de la température servant à réguler la température de parties cutanées du corps humain

(30) Priorität: 11.10.2007 WO PCT/EP2007/008829
(43) Veröffentlichungstag der Anmeldung: 16.06.2010
(73) Patentinhaber: Hilotherm Holding AG, 6317 Oberwil b. zug (CH)
(72) Erfinder: JANISCH, Klaus, 88316 Isny (DE)
(74) Vertreter: Clerc, Natalia
(86) Internationale Anmeldenummer: PCT/EP2008/003747
(87) Internationale Veröffentlichungsnummer: WO 2009/049688

(56) Entgegenhaltungen:
- DE-A1- 2 722 613
- DE-U1- 29 716 338
- US-A- 4 821 354
- US-A1- 2003 163 179

## Beschreibung

Die Erfindung betrifft eine Temperiereinrichtung zum Temperieren von Körperstellen des menschlichen Körpers, mit einem Temperierkissen, das mit einem Temperiergerät koppelbar, am Körper anlegbar und von temperiertem Fluid durchströmbar ist, mit wenigstens zwei Schlauchanschlussöffnungen, in denen jeweils ein Schlauch-Ende eines Versorgungsschlauchs einer Schlauchanordnung angeordnet oder einführbar ist, über die temperiertes Fluid im Kreislauf zwischen Temperiereinheit und Temperierkissen transportierbar ist, wobei temperiertes Fluid jeweils über einen Durchströmquerschnitt in das Temperierkissen oder aus dem Temperierkissen gelangt.

Aus der DE 296 23 224 U1 ist eine Temperiereinheit bekannt, bei der wenigstens ein Temperierkissen an ein Versorgungsgerät angeschlossen werden kann, das seinerseits einen wärmeisolierten Behälter zur Aufnahme von zu temperierender Flüssigkeit, insbesondere Wasser, besitzt. Mit dem Versorgungs- bzw. Temperiergerät ist eine konstante Temperatur der temperierten Flüssigkeit einstellbar, so dass beispielsweise mit konstanter Temperatur gekühlt werden kann. Dies hat bedeutende Vorteile gegenüber herkömmlichen Kühlmethoden mittels Eiswürfeln oder mittels flüssigkeitsgefüllter Kühlkissen, da im erstgenannten Fall oftmals mit einer zu niedrigen Temperatur gekühlt wird, was zu lokalen Erfrierungen führen kann. Flüssigkeitsgefüllte Kühlkissen erwärmen sich hingegen relativ schnell, so dass eine Kühlung mit konstanter Temperatur nicht möglich ist.

Je nach zu temperierender Körperstelle des menschlichen Körpers sind Temperierkissen unterschiedlichster Ausgestaltung einsetzbar. Beispielsweise gibt es Temperierkissen, die zur Kühlung am Kopf, beispielsweise im Gesichtsbereich, eingesetzt werden, oder solche, die zur Kühlung bestimmter Korpuspartien verwendet werden. Um einen Kühl- bzw. Wärmeeffekt mit konstanter Temperatur zu erzielen, ist es notwendig, dass ein zuverlässiger Austausch an temperierter Flüssigkeit im Temperierkissen stattfindet. Die temperierte Flüssigkeit gelangt über einen Zulaufschlauch in das Temperierkissen und wird dort über ein Fluidkanalsystem aus labyrinth- oder mäanderförmigen Fluidkanälen über das gesamte Temperierkissen verteilt zu einer Auslassöffnung geführt und gelangt dort über einen Rücklaufschlauch wieder zum Temperiergerät, wo es wieder auf die entsprechende Temperatur gebracht wird.

Damit sich das Temperierkissen an die zu temperierende Körperstelle gut anpassen kann, besteht dieses aus flexiblem Material. Dabei kann es vorkommen, dass es zu einem Abknicken im Bereich der Schlauch-Enden der Versorgungsschläuche kommt, beispielsweise dadurch, dass das Temperierkissen verrutscht und der Patient ungünstig darauf zu liegen kommt. In diesem Fall kann es vorkommen, dass der Durchströmquerschnitt für das temperierte Fluid vollständig verschlossen wird, was dazu führt, dass der Fluidaustausch im Temperierkissen zum Erliegen kommt, wodurch ein Kühl- bzw. Wärmeeffekt mit konstanter Temperatur nicht mehr möglich ist.

DE 27 22 613 offenbart eine flexibles thermisches Kissen mit einer fluiddurchströmten Kammer und einem Fluideinlasse und einem Fluidauslass. In diese Öffnungen lassen sich Schläuche einstecken. Um ein Abknicken des Schlauchs im Bereich dieser Öffnungen zu verhindern, ist dieses Ende des Schlauchs mit einer v-förmigen Kerbe versehen oder der Schlauch weist ein diskontinuierliches Ende auf.

US 2003/0163179 beschreibt ein thermisches Polster mit spitzenartigen Schlauchenden.

DE 297 16 338 zeigt eine Temperiermanschette mit einem Knickschutz in Form einer flachen, permeablen Kunststoffeinlage. Die Schläuche ragen in diese Einlage hinein und sind in diesem Bereich perforiert.

Auch US 4 821 354 und DE 296 23 224 offenbaren Temperiereinrichtungen.

Aufgabe der Erfindung ist es, eine Temperiereinrichtung der eingangs erwähnten Art zu schaffen, mit der das Temperieren von Körperstellen des menschlichen Körpers noch zuverlässiger möglich ist.

Diese Aufgabe wird durch eine Temperiereinrichtung mit den Merkmalen des unabhängigen Anspruchs 1 gelöst. Weiterbildungen der Erfindung sind in den Unteransprüchen dargestellt.

Die erfindungsgemäße Temperiereinrichtung zeichnet sich dadurch aus, dass Aufhaltemittel vorgesehen sind, die den Durchströmquerschnitt bei Abknicken des Temperierkissens im Bereich der Schlauch-Enden zumindest teilweise offen halten.

Die Aufhaltemittel sorgen also dafür, dass es bei Abknicken des Temperierkissens, das auch als Manschette bezeichnet werden kann, zu keinem vollständigen Verschließen des Durchströmquerschnitts kommt. Dadurch bleibt der Austausch an temperiertem Fluid im Temperierkissen erhalten, so dass weiterhin ein zuverlässiges Temperieren möglich. Beispielsweise wird bei einem für kühlzwecke verwendeten Temperierkissen verhindert, dass sich das im Temperierkissen befindliche temperierte Fluid zu stark erwärmt.

Bei einer Weiterbildung der Erfindung sind die Aufhaltemittel derart ausgebildet, dass eine Biegeflexibilität des Temperierkissens im Bereich der Schlauch-Enden erhalten bleibt. Dadurch ist gewährleistet, dass dem Temperierkissen auch im Bereich der Schlauch-Enden eine wichtige Eigenschaft erhalten bleibt, nämlich sich an die zu temperierenden Körperstellen anschmiegen zu können.

In besonders bevorzugter Weise sind die Aufhaltemittel den Schlauch-Enden der Versorgungsschläuche zugeordnet. Bei den Versorgungsschläuchen handelt es sich um wenigstens einen Zulaufschlauch für in das Temperierkissen zu transportierendes temperiertes Fluid und wenigstens einen Rücklaufschlauch zum Rücktransport an temperiertem Fluid in das Versorgungs- bzw. Temperiergerät.

Bei einer Weiterbildung der Erfindung weisen die Aufhaltemittel wenigstens einen von einer Stirnseite des Schlauch-Endes in Axialrichtung verlaufenden, in die Schlauchwandung eingebrachten Aufhalteausschnitt auf.

Es ist möglich, dass wenigstens zwei Aufhalteausschnitte vorgesehen sind. Bei genau zwei Aufhalteausschnitten können diese diametral zueinander angeordnet sein. Bei mehr als zwei Aufhalteausschnitten können diese gleichmäßig über den Umfang verteilt angeordnet sein.

Besonders bevorzugt ist der wenigstens eine Aufhalteausschnitt V- und/oder U-artig ausgestaltet. Es sind jedoch auch andere Ausschnittsgeometrien denkbar.

Bei einer Weiterbildung der Erfindung weisen die Aufhaltemittel einen von der Stirnseite des Schlauch-Endes nach vorne abragenden Aufhalteansatz auf. Der Aufhalteansatz kann also fingerartig vom Schlauch-Ende nach vorne abstehen.

Zweckmäßigerweise ist der Aufhalteansatz einstückig mit dem Schlauch-Ende verbunden. Bevorzugterweise wird der Aufhalteansatz von einem Abschnitt der Schlauchwandung gebildet. Dadurch lässt sich der Aufhalteansatz relativ einfach herstellen.

Gemäß der Erfindung weisen die Aufhaltemittel wenigstens ein in den zugeordneten Versorgungsschlauch einlegbares, längliches Einlegeprofil auf, das im eingelegten Zustand von temperiertem Fluid umströmbar ist und von der Stirnseite des Schlauch-Endes mit Überstand nach vorne abragt. Im eingelegten Zustand kann sich der Überstandsbereich des Einlegeprofils ein gutes Stück in das Fluidkanalsystem des Temperierkissens hinein erstrecken. In besonders bevorzugter weise besteht das Einlegeprofil aus biegeflexiblem Material. Dadurch lässt sich das Einlegeprofil auch an Biegungen im Fluidkanalsystem anpassen.

Damit das Einlegeprofil nicht in Schlauch-Längsrichtung verrutscht, sind Fixiermittel zur axialen Fixierung der gewünschten Einlegeposition des Einlegeprofils vorgesehen. Besonders bevorzugt werden die Fixiermittel vom Einlegeprofil selber gebildet.

Zweckmäßigerweise besitzen die Fixiermittel wenigsten einen, von einem Außenumfang des Einlegeprofils nach außen abstehenden Fixiervorsprung, der sich zur Vorgabe einer axialen Fixierposition des Einlegeprofils im Schlauch an der Stirnseite des Schlauch-Endes widerhakenartig abstützt.

Besonders bevorzugt ist der Fixiervorsprung von einer biegeflexiblen Fixierzunge gebildet, die beim Einführen des Einlegeprofils durch ein Innenmaß des Versorgungsschlauches federnd zusammendrückbar ist und nach Passieren des Schlauch-Endes federnd aufschnappt. Das Einlegeprofil lässt sich also insbesondere mittels eines geeignetem Einführwerkzeuges von der einen seite des Schlauches aus ein- und durch den Versorgungsschlauch hindurchführen, wobei das Einlegeprofil dann am anderen Schlauch-Ende austritt und die Fixiervorsprünge, insbesondere die aufschnappenden Fixierzungen dafür sorgen, dass das Einlegeprofil axial fixiert ist und nicht mehr entgegen der Einführrichtung verrutschen kann. Alternativ ist es auch möglich, die Fixiervorsprünge als starre Elemente auszubilden, die dennoch bei geeignetem Schlauchmaterial durch Aufweitung des Schlauches durch das Schlauchinnere passen, wobei sich der Schlauch dann wieder beim Austritt des Fixiervorsprungs auf seine ursprüngliche Weite verengt.

Besonders zweckmäßig sind mehrere Fixiervorsprünge, insbesondere biegeflexible Fixierzungen, in Längsrichtung des Einlegeprofils hintereinander angeordnet. Beispielsweise ist es möglich, dass wenigstens zwei Reihen mit jeweils hintereinander angeordneten Fixiervorsprüngen vorgesehen sind, wobei vorzugsweise die Fixiervorsprünge der einen Reihe in Längsrichtung versetzt zu den Fixiervorsprüngen der anderen Reihe angeordnet sind. Zweckmäßigerweise befindet sich die eine Reihe mit Fixiervorsprüngen an der einen Seite des Einlegeprofils und die andere Reihe an der gegenüberliegenden Seite. Zweckmäßigerweise befindet sich ein jeweiliger Fixiervorsprung der einen Reihe auf Höhe einer Lücke zwischen zwei hintereinander liegenden Fixiervorsprüngen der anderen Reihe.

Eine andere Möglichkeit ist, die Fixiermittel durch eine wellenartige Ausgestaltung des Einlegeprofils zu bilden, so dass das Einlegeprofil federartig in den zugeordneten Versorgungsschlauch eingespannt werden kann.

Es ist möglich, das Einlegeprofil als Hohlprofil auszugestalten, wodurch temperiertes Fluid auch über das Profil-Innere in das Temperierkissen bzw. aus dem Temperierkissen heraus gelangen kann. Jedoch sind auch eine Vielzahl anderer Querschnitts-Geometrien des Einlegeprofils einsetzbar, die ein Umströmen mit temperiertem Fluid zulassen.

Bei einer Weiterbildung der Erfindung weist das Einlegeprofil einen Einführabschnitt mit gegenüber dem Rest des Einlegeprofils größerem Querschnitt zum Einführen in den zugeordneten Versorgungsschlauch bzw. in die zugeordnete Schlauchanschlussöffnung des Temperierkissens auf. Die Form des Einführabschnittes ist dabei so ausgestaltet, dass ein einfaches Einführen des Einlegeprofils in den zugeordneten Versorgungsschlauch möglich ist. Der Einführabschnitt kann beispielsweise kugel- oder ovalförmig ausgestaltet sein.

Zweckmäßigerweise besitzt das Einlegeprofil einen insbesondere in Profil-Längsrichtung an den Einführabschnitt anschließenden, biegeflexiblen Biegeabschnitt, mit dem eine Führung des Einlegeprofils in den Biegungen der Kanalordnung möglich ist.

Bei einer Weiterbildung der Erfindung weist das Einlegeprofil einen insbesondere in Profil-Längsrichtung an den Biegeabschnitt anschließenden, stegartig ausgebildeten Basisabschnitt auf, an dem der wenigstens eine Fixiervorsprung sitzt.

Bei einer Weiterbildung der Erfindung sind die Schlauchanschlussöffnungen Bestandteil eines im Temperierkissen angeordneten Schlauchanschlussstücks, an dem sich die Aufhaltemittel befinden oder dem die Aufhaltemittel zugeordnet sind.

Die Temperiereinrichtung kann ferner noch das vorstehend bereits erwähnte Temperiergerät umfassen.

Bevorzugte Ausführungsbeispiele der Erfindung sind in der Zeichnung dargestellt und werden im Folgenden näher erläutert. In den Zeichnungen zeigen:
- Fig. 1: eine Draufsicht auf ein Temperierkissen gemäß einem ersten Ausführungsbeispiel der erfindungsgemäßen Temperiereinrichtung,
- Fig. 2: Seitenansichten der Schlauch-Enden des Versorgungs- schlauchs von Figur 1 in vier verschiedenen Ausfüh- rungsformen A bis D, wobei paarweise 90° zueinander versetzte Seitenansichten gezeigt sind,
- Fig. 3: eine teilweise geschnittene Draufsicht auf ein Tem- perierkissen gemäß einem zweiten Ausführungsbei- spiel der erfindungsgemäßen Temperiereinrichtung,
- Fig. 4: Seitenansichten der Schlauch-Enden der Versorgungs- schläuche von Figur 3, wobei Figur 4 **I** und Figur 4 II 90° versetzt zueinander liegende Seitenansich- ten zeigen,
- Fig. 5: eine teilweise geschnittene Seitenansicht auf ein Temperierkissen gemäß einem dritten Ausführungsbei- spiel der erfindungsgemäßen Temperiereinrichtung,
- Fig. 6: Vorder- bzw. Seitenansichten der Einlegeprofile von Figur 5 in fünf verschiedenen Ausführungsformen A bis E, wobei durch I die Vorder- und durch II die Seitenansichten bezeichnet sind,
- Fig. 7: eine teilweise geschnittene Draufsicht auf ein Tem- perierkissen eines vierten Ausführungsbeispiels der erfindungsgemäßen Temperiereinrichtung,
- Fig. 8: Seitenansichten (I) und Vorderansichten (II) der Schlauchanschlussstücke von Figur 7 in zwei ver- schiedenen Ausführungsformen A und B,
- Fig. 9: eine teilweise geschnittene Draufsicht auf ein Tem- perierkissen gemäß einem fünften Ausführungsbei- spiel der erfindungsgemäßen Temperiereinrichtung,
- Fig. 10: einen Längsschnitt durch einen der Versorgungs- schläuche von Fig. 9 mit eingelegtem Einlegeprofil,
- Fig. 11: einen Schnitt durch den Versorgungsschlauch und das Einlegeprofil gemäß der Linie XI-XI von Fig. 10,
- Fig. 12: einen Längsschnitt durch einen der Versorgungs- schläuche mit eingelegtem Einlegeprofil in gegen- über Fig. 10 90° gedrehter Ansicht und
- Fig. 13: einen Querschnitt durch den Versorgungsschlauch und das Einlegeprofil entlang der Linie XIII-XIII von Fig. 12.

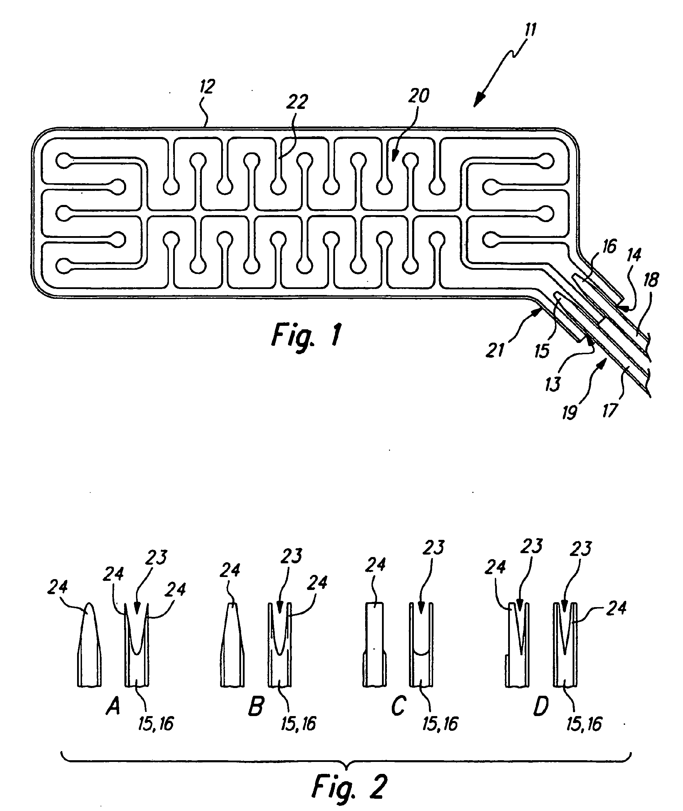

Figur 1 zeigt ein erstes Ausführungsbeispiel einer Temperiereinrichtung 11. Die Temperiereinrichtung 11 dient zum Temperieren von Körperstellen des menschlichen oder auch tierischen Körpers. Sie kann sowohl zum Kühlen als auch zum Wärmen eingesetzt werden. Bei Verwendung als Kühlapplikation hat die Temperiereinrichtung 11 gegenüber den bereits vorstehend erwähnten herkömmlichen Kälteanwendungen wie Eiswürfel und flüssigkeitsgefülltem Kühlkissen immense Vorteile. Die konventionellen Applikationen lassen keine exakte Temperatursteuerung zu, können also Gewebeschäden oder Nervenlähmungen durch zu niedrige Kühltemperatur verursachen. Mit der Temperiereinrichtung ist eine Kühlung mit konstanter Temperatur möglich, so dass sie sich hervorragend zur Schwellungs- und Schmerzprophylaxe eignet.

Die Temperiereinrichtung besitzt ein Temperierkissen 12, das mit einem Temperiergerät (nicht dargestellt) koppelbar, am menschlichen bzw. tierischen Körper anlegbar und von temperiertem Fluid durchströmbar ist. Ferner sind wenigstens zwei Schlauchanschlussöffnungen 13, 14 vorgesehen, in denen jeweils ein Schlauch-Ende 15, 16 eines Versorgungsschlauchs 17, 18 einer Schlauchanordnung 19 angeordnet oder einführbar ist, über die temperiertes Fluid im Kreislauf zwischen Temperiergerät und Temperierkissen 12 transportierbar ist.

Die Temperiereinrichtung 11 kann ferner noch das vorstehend bereits erwähnte Temperiergerät aufweisen, wobei dann zum Gesamtsystem auch mehrere Temperierkissen 12 gehören können. Das heißt, über das Temperiergerät sind mehrere Temperierkissen mit temperiertem Fluid versorgbar.

Je nach zu temperierender Körperpartie kann das Temperierkissen 12 unterschiedlichste Ausgestaltungen aufweisen. Je nach geometrischer Ausgestaltung eignet sich das Temperierkissen 12 zum Auflegen auf entsprechende Bereiche von Kopf, Körper und Gliedmaßen. Temperierkissen sind z. B. einsetzbar zum Auflegen im Stirn-, Nacken-, Augen-, Kieferbereich, an Schulter, Hand, Brust, Hüfte, Knie, Wade, Fuß und allen Körperpartien bei denen eine Thermotherapie angezeigt ist.

Das Temperierkissen 12 besteht aus Kunststoff. Beispielsweise kann PU verwendet werden, womit das Temperierkissen 12 kostengünstig herstellbar ist, so dass beispielsweise auch ein Einmal-Gebrauch möglich ist.

Als temperiertes Fluid wird eine Flüssigkeit, insbesondere Wasser, verwendet. Wie insbesondere in den Figuren 1, 3, 5 und 7 gezeigt, besitzt das Temperierkissen 12 eine Kanalanordnung 20, die von einer als Einlassöffnung ausgebildeten Schlauchanschlussöffnung 13 gleichmäßig über das Temperierkissen 12 verteilt zu einer als Auslassöffnung ausgebildeten Schlauchanschlussöffnung 14 führt. Dadurch ist gewährleistet, dass temperiertes Fluid gleichmäßig in alle Bereiche des Temperierkissens 12 gelangt. Das Temperierkissen 12 besitzt ferner noch eine Schlauchanschlusspartie 21, an der sich die Schlauchanschlussöffnungen 13, 14, also die Einlass- bzw. Auslassöffnung, befinden. Selbstverständlich ist es auch möglich, mehr als zwei Schlauchanschlussöffnungen vorzusehen, beispielsweise zwei Einlassöffnungen und zwei Auslassöffnungen.

Wie insbesondere in Figur 1 dargestellt, befindet sich in der Einlassöffnung ein Schlauch-Ende 15 eines als Zulaufschlauch ausgebildeten Versorgungsschlauchs 17. In der Auslassöffnung befindet sich ein Schlauch-Ende 16 eines als Rücklaufschlauch ausgebildeten Versorgungsschlauchs 18. Gemäß erstem Ausführungsbeispiel sind die Versorgungsschläuche 17, 18 unlösbar mit dem Temperierkissen 12 verbunden, insbesondere werden sie bei der Herstellung des Temperierkissens gleich in die entsprechenden Schlauchanschlussöffnungen 13, 14 mit eingeschweißt. Die Herstellung des Temperierkissens 12 erfolgt bevorzugterweise derart, dass zwei Lagen aus Kunststoffmaterial mit gleichen Außenabmessungen übereinandergelegt und an bestimmten Schweißnähten 22 zusammengeschweißt werden, wobei je nach Verlauf der Schweißnähte 22 ein charakteristischer, beispielsweise mäanderartiger Verlauf der Kanalanordnung 20 vorgegeben werden kann. Bei der Herstellung werden die Schlauch-Enden 15, 16 der Versorgungsschläuche 17, 18 zwischen die beiden Lagen gebracht und mit eingeschweißt.

Bei herkömmlichen Temperierkissen besitzen die Schlauch-Enden 15, 16 einen geraden Abschluss, wodurch eine relativ scharfe Kante am Übergang zwischen den Schlauch-Enden 15, 16 und dem Temperierkissen entsteht. In der Regel ist das Material des Temperierkissens weicher und biegeflexibler als das Material der Versorgungsschläuche. Bei einer derartigen aus dem Stand der Technik bekannten Ausgestaltung der Schlauch-Enden 15, 16 kann es vorkommen, dass das Temperierkissen 12 im Bereich der Schlauch-Enden 15, 16, insbesondere direkt am Übergang zwischen dem Schlauch-Ende und dem Temperierkissen, abknickt und dadurch ein Durchströmquerschnitt, über den temperiertes Fluid in das Temperierkissen 12 einströmt oder aus diesem ausströmt, vollständig verschlossen wird. Dies hat zur Folge, dass der Austausch an temperiertem Fluid zum Erliegen kommt und eine Kühl- bzw. Wärmeanwendung mit konstanter Temperatur nicht mehr möglich ist.

Um das vollständige Verschließen des Durchströmquerschnitts zu verhindern, sind Aufhaltemittel vorgesehen, die den Durchströmquerschnitt bei Abknicken des Temperierkissens 12 im Bereich der Schlauch-Enden 15, 16 zumindest teilweise offen halten. Gemäß erstem Ausführungsbeispiel weisen die Aufhaltemittel wenigstens einen Aufhalteausschnitt 23 auf, der von einer Stirnseite des Schlauch-Endes 15, 16 in Axialrichtung verlaufend in die Schlauchwandung eingebracht ist. Die Aufhalteausschnitte können in einfacher Weise mit einem geeigneten Schneidwerkzeug, insbesondere Messer, in die Schlauchwandung eingeschnitten werden. Somit sind diese Aufhalteausschnitte in einfacher Weise herstellbar.

Gemäß Figur 2A sind zwei Aufhalteausschnitte 23 vorgesehen, die sich diametral gegenüberliegen, so dass zwei einander gegenüberliegende Lappen 24 übrig bleiben. Gemäß Figur 2A sind die Aufhalteausschnitte 23 V-artig ausgestaltet und weisen am Grund eine Rundung auf. Die Schnittiefe kann variabel gestaltet werden. Bei der in Figur 2A gezeigten Ausführungsform besitzen die Aufhalteausschnitte 23 im Bereich der Stirnseiten der Schlauch-Enden 15, 16 eine relativ große Breite, so dass die Lappen relativ dünnwandig und somit sehr biegeflexibel ausgestaltet sind.

Wie in der Ausführungsform 2B beispielhaft dargestellt, können auch zwei V-artige Aufhalteausschnitte 23 vorgesehen sein, die im vergleich zur Ausführungsform 2A schmaler ausgestaltet sind, so dass die übrig bleibenden Lappen 24 eine etwas größere Dicke bzw. Stärke aufweisen. Bevorzugterweise ist auch hier eine Rundung am Grund vorgesehen.

Wie in der Ausführungsform 2C beispielhaft dargestellt, können die Aufhalteausschnitte 23 auch eine U-artige Gestalt besitzen, wobei bevorzugterweise auch hier eine Rundung am Grund vorgesehen sein kann. Es bleiben hier dann zwei in Draufsicht rechteckförmige Lappen 24 übrig.

Schließlich zeigt die Ausführungsform gemäß Figur 2D das Anbringen von mehr als zwei Aufhalteausschnitten 23, beispielsweise drei an der Zahl, die selbstverständlich auch U- oder V- oder andersartig geometrisch ausgestaltet sein können.

Beim Abknicken des Temperierkissens 12 im Bereich der Schlauch-Enden 15, 16 werden gemäß dem ersten Ausführungsbeispiel die überstehenden Lappen 24 mit umgebogen, und zwar beide in eine Richtung. Der Durchströmquerschnitt für das temperierte Fluid verschließt sich beim Abknicken jedoch nicht vollständig, da temperiertes Fluid nach wie vor über die Aufhalteausschnitte seitlich an den Lappen 24 vorbeiströmen kann.

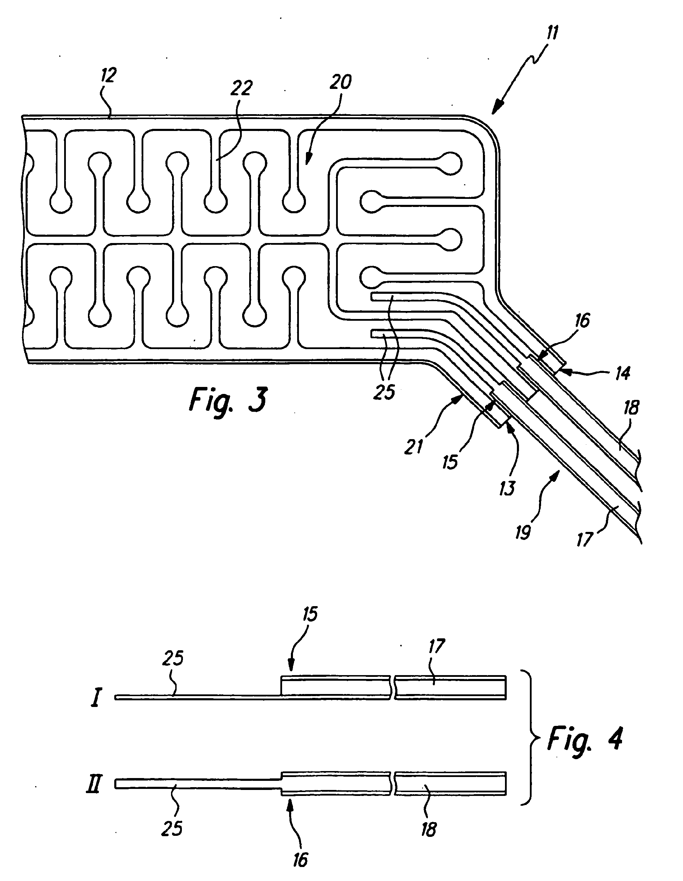

Die Figuren 3 und 4 zeigen ein zweites Ausführungsbeispiel einer Temperiereinrichtung 11. Es unterscheidet sich von dem zuvor beschriebenen Ausführungsbeispiel durch die andersartige Ausgestaltung der Aufhaltemittel. Die Aufhaltemittel weisen hier einen von der Stirnseite des Schlauch-Endes 15, 16 nach vorne abragenden, fingerartigen Aufhalteansatz 25 auf. Der Aufhalteansatz 25 wird von der Schlauchwandung selber gebildet, und zwar dadurch, dass die um den fingerartigen Aufhalteansatz 25 angrenzenden Bereiche der Schlauchwandung einfach abgetrennt, insbesondere weggeschnitten sind. Die Länge des Aufhalteansatzes 25 ist variabel und kann, wie beispielhaft in Figur 3 dargestellt, derart bemessen sein, dass der Aufhalteansatz um Biegungen der Kanalanordnung 20 herumführbar ist.

Beim Abknicken des Temperierkissens 12 im Bereich der Schlauch-Enden 15, 16 biegt sich auch der fingerartige Aufhalteansatz durch. Da das Material der Versorgungsschläuche 17, 18 jedoch steifer ist als das Material des Temperierkissens 12, führt dies dazu, dass der umgebogene fingerartige Aufhalteansatz 25 den Durchströmquerschnitt nicht vollständig verschließt, sondern ebenfalls zumindest eine schmale Öffnung offen bleibt, über die temperiertes Fluid ein- bzw. ausströmen kann.

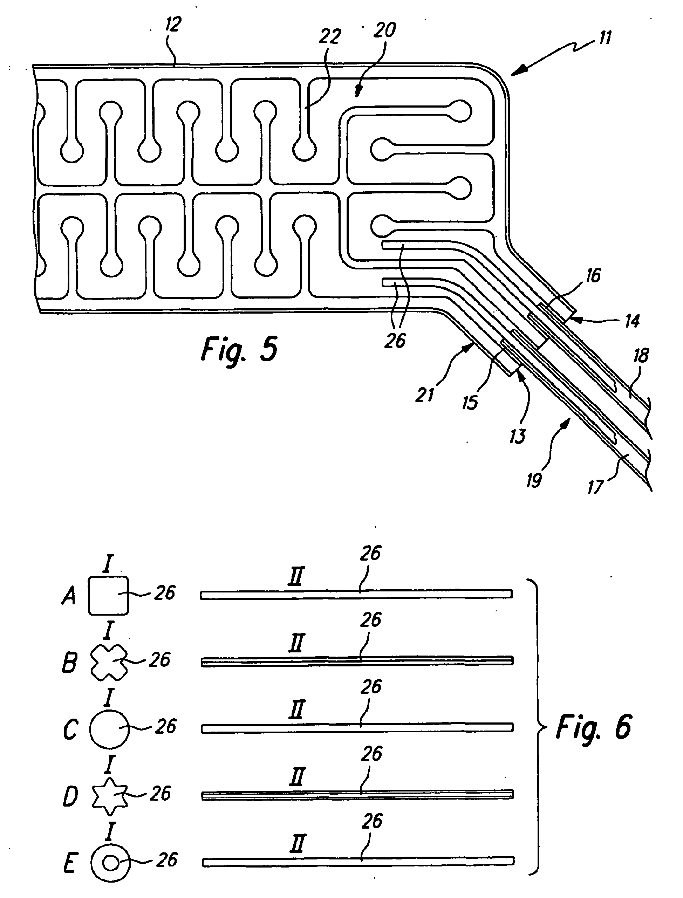

Die Figuren 5 und 6 zeigen ein Ausführungsbeispiel der erfindungsgemäßen Temperiereinrichtung 11. Dieses Ausführungsbeispiel unterscheidet sich von den zuvor bereits beschriebenen Ausführungsbeispielen ebenfalls durch die andersartige Ausgestaltung der Aufhaltemittel. Die Aufhaltemittel besitzen hier wenigstens ein in den zugeordneten Versorgungsschlauch 17, 18, also in den Zulauf- bzw. Rücklaufschlauch, einlegbares, längliches Einlegeprofil 26, das im eingelegten Zustand von temperiertem Fluid umströmbar ist und von der Stirnseite des Schlauch-Endes 15,16 mit Überstand nach vorne abragt. Das Einlegeprofil 26 kann in einfacher Weise über die freien Schlauch-Enden 15, 16 gegebenenfalls mit einem geeigneten Einschiebemittel in die gewünschte Einlegeposition eingeschoben werden. Das Einlegeprofil 26 besteht aus biegeflexiblem Material, so dass es ähnlich wie beim Aufhalteansatz 25 des zweiten Ausführungsbeispiels möglich ist, das Einlegeprofil 26 um Biegungen der Kanalanordnung 20 herumzuführen.

Wie in Figur 6 dargestellt, sind verschiedene Querschnitts-Geometrien des Einlegeprofils 26 denkbar. Voraussetzung hierfür ist, dass trotz eingelegtem Einlegeprofil 26 noch genügend temperiertes Fluid ein- bzw. ausströmen kann. Gemäß Figur 6A ist ein Einlegeprofil 26 mit quadratischem Querschnitt denkbar, wobei dann zwischen den Außenflächen des Einlegeprofils und der Innenwand des Versorgungsschlauches 17, 18 Strömungskanäle gebildet sind, über die das temperierte Fluid strömt.

Bei der Ausführungsform gemäß Figur 6B mit kreuzförmigem Querschnitt und der Ausführungsform gemäß Figur 6D mit sternförmigem Querschnitt werden ebenfalls Strömungskanäle zwischen der Innenwand des Schlauches und der Außenwand des Einlegeprofils 26 gebildet.

Bei der Ausführungsform gemäß Figur 6C muss dafür gesorgt werden, dass der Außendurchmesser des Einlegeprofils 26 ausreichend kleiner als der Innendurchmesser des Versorgungsschlauchs ausgebildet ist. Wie beispielhaft in der Ausführungsform gemäß Figur 6E gezeigt, kann das Einlegeprofil 26 auch als Hohlprofil ausgebildet sein, wobei dann temperiertes Fluid über das Profil-Innere ein- bzw. ausströmen kann. Selbstverständlich ist es möglich, eine ganze Reihe anderer Querschnitts-Geometrien des Einlegeprofils 26 einzusetzen.

Damit das Einlegeprofil 26 in seiner gewünschten Einlegeposition nicht verrutscht, können Fixiermittel zur axialen Fixierung des Einlegeprofils 26 vorgesehen sein. Bei den Ausführungsformen gemäß Figur 6A, 6B und 6D ist ein passgenauer Sitz des Einlegeprofils 26 im Versorgungsschlauch 17, 18 möglich, da temperiertes Fluid durch die vorstehend erwähnten Strömungskanäle zwischen der Außenwand des Einlegeprofils 26 und der Innenwand des Versorgungsschlauchs 17, 18 strömen kann. Auch bei der Ausführungsform gemäß Figur 6E ist ein passgenauer Sitz möglich, da temperiertes Fluid durch das Profil-Innere strömen kann. Bei der Ausführungsform gemäß Figur 6C ist es notwendig, dass das Einlegeprofil mit Spiel im Versorgungsschlauch sitzt, damit temperiertes Fluid vorbeiströmen kann. Insbesondere dort ist es möglich, Fixiermittel vorzusehen, die von einer in Längsrichtung wellenartigen Ausgestaltung des Einlegeprofils gebildet sind, wodurch das Einlegeprofil federartig in den Versorgungsschlauch eingespannt werden kann.

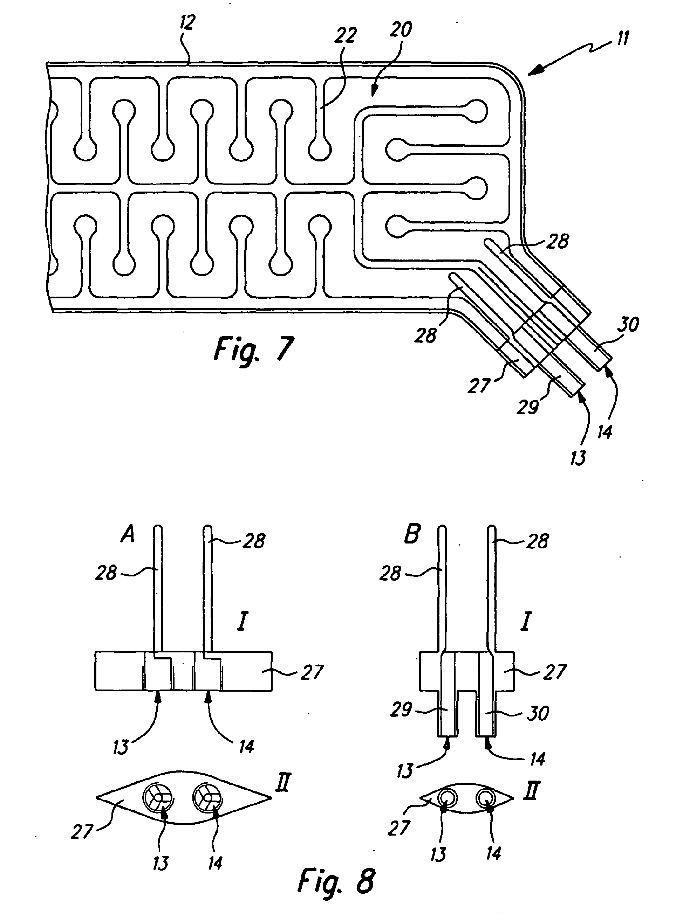

Die Figuren 7 und 8 zeigen ein viertes Ausführungsbeispiel einer Temperiereinrichtung 11. Die Schlauchansschlussöffnungen 13, 14 sind hier Bestandteil eines im Temperierkissen 12 angeordneten Schlauchanschlussstücks 27, an dem sich die Aufhaltemittel befinden oder dem die Aufhaltemittel zugeordnet sind. Im Gegensatz zu den vorstehend beschriebenen Ausführungsbeispielen, bei denen die Versorgungsschläuche 17, 18 in das Temperierkissen 12 eingeschweißt sind, können die Versorgungsschläuche 17, 18 hier lösbar an das Schlauchanschlussstück 27 angekuppelt werden.

Wie beispielhaft in Figur 8A dargestellt, können die beiden Schlauchanschlussöffnungen 13, 14 in dem Schlauchanschlussstück 27 ausgebildet und jeweils mit einem Gewinde versehen sein, in das ein mit einem Gegengewinde versehenes Schlauch-Ende eingeschraubt werden kann. Als Aufhaltemittel dient hier ähnlich wie beim zweiten Ausführungsbeispiel ein fingerartig vom Schlauchanschlussstück 27 nach vorne abragender Aufhalteansatz 28. Prinzipiell ist es jedoch auch möglich, eine Kombination von Schlauchanschlussstücken 27 und Aufhaltemitteln in Form von Einlegeprofilen 26 zu verwenden.

Eine Alternative ist in Figur 8B dargestellt, wobei hier zwei über die Außenabmessung des Temperierkissens 12 hinausragende Anschlusshülsen 29, 30 vorgesehen sind, in die die Schlauch-Enden 15, 16 der Versorgungsschläuche 17, 18 eingesteckt werden können. Auch hier sind Aufhaltemittel in Form von fingerartigen Aufhalteansätzen 28 vorgesehen.

Die Schlauchanschlussstücke 27 sind in bevorzugter Weise in das Temperierkissen 12 eingeschweißt.

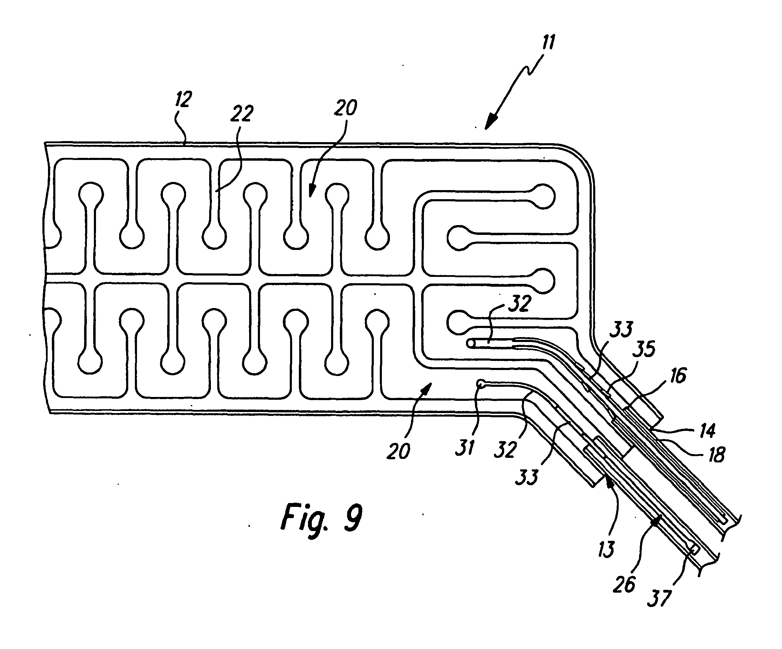

Die Fig. 9 bis 13 zeigen ein fünftes Ausführungsbeispiel. Es unterscheidet sich von den zuvor beschriebenen Ausführungsbeispielen ebenfalls durch die andersartige Ausgestaltung der Aufhaltemittel. Es ist hier wiederum, wie bereits beim dritten Ausführungsbeispiel gezeigt, ein am Zulauf- bzw. Rücklaufschlauch fixierbares, längliches Einlegeprofil 26 vorgesehen, das im eingelegten Zustand von temperiertem Fluid umströmbar ist und von der Stirnseite des Schlauch-Endes 15, 16 mit Überstand nach vorne abragt. Das Einlegeprofil 26 kann prinzipiell in drei Abschnitte eingeteilt werden. Es ist ein vordere Einführabschnitt 31 vorgesehen, der gegenüber dem Rest des Einlegeprofils 26 einen größeren Querschnitt besitzt. Der Einführabschnitt 31 ist hier beispielhaft in Form eines Kugelkopfes dargestellt. Es sind jedoch auch andere Geometrien denkbar. Der Einführabschnitt 31 sorgt zum einen dafür, dass das Einlegeprofil 26 in einfacher Weise in den zugeordneten Versorgungsschlauch 17, 18 eingeführt und durch diesen in nachfolgend noch näher beschriebenen Weise hindurchführbar ist, bis ein Teil des Einlegeprofils 26 mit Überstand aus dem zugeordneten Versorgungsschlauch 17, 18 hervorsteht. Zum anderen sorgt der Einführabschnitt 31 für ein leichtes Einführen in die zugeordneten Schlauchanschlussöffnungen 13, 14 am Temperierkissen 12.

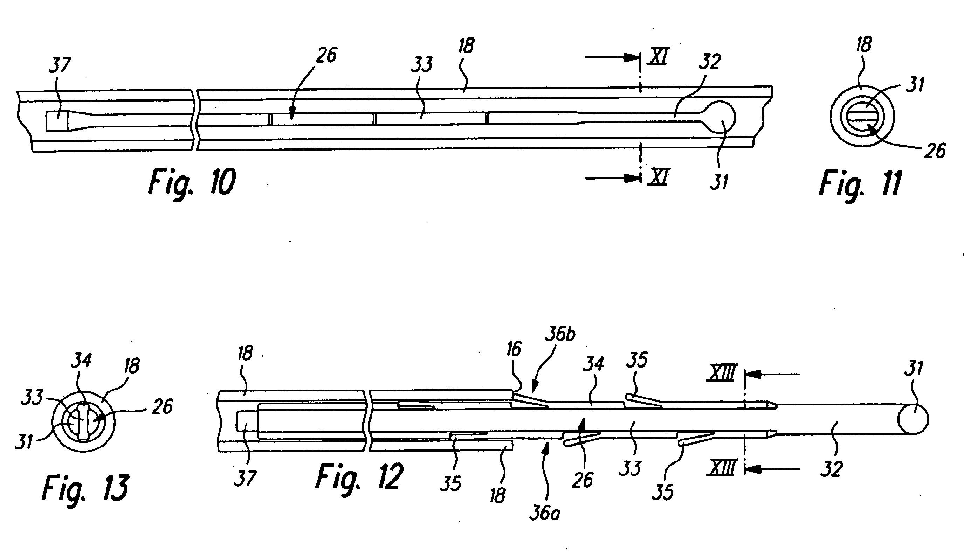

In Einführrichtung hinter dem Einführabschnitt befindet sich ein biegeflexibler Biegeabschnitt 32, mit gegenüber dem Einführabschnitt 31 kleinerem Querschnitt. Der Biegeabschnitt 32 ist, wie insbesondere in Fig. 10 dargestellt, beispielhaft in flachprofilartiger Ausgestaltung dargestellt. Der Biegeabschnitt 32 sorgt dafür, dass das Einlegeprofil 26 auch um Biegungen der Kanalanordnung 20 herumgeführt werden kann.

In Einführrichtung hinter dem Biegeabschnitt 32 ist ein Basisabschnitt 33 angeordnet, der wie in den Fig. 10 und 12 dargestellt, den Großteil des Einlegeprofils 26 einnimmt. Auch der Basisabschnitt 33 ist wie der Biegeabschnitt 32 zweckmäßigerweise flachprofilartig bzw. stegförmig ausgestaltet und besitzt bevorzugterweise einen doppel-T-artigen Querschnitt, wobei sich an den beiden entgegengesetzten Schmalseiten des Basisabschnitts 33 Verdickungen 34 befinden. Am Basisabschnitt 33 befinden sich die Fixiermittel in Form wenigstens eines vom Außenumfang des Einlegeprofils 26 nach außen abstehenden Fixiervorsprungs 35 in Form einer Fixierzunge. Die Fixierzunge stützt sich zur Vorgabe einer axialen Fixierposition des Einlegeprofils 26 im Schlauch an der Stirnseite des Schlauch-Endes 15, 16 widerhakenartig ab. Es sind hier mehrere in Profil-Längsrichtung des Einlegeprofils 26 hintereinander angeordnete Fixierzungen vorgesehen, die im Bereich der Verdickungen an den Schmalseiten des Basisabschnitts 33 angeordnet sind und von dort nach außen abragen. Die Fixierzungen sind biegeflexibel ausgestaltet und können beim Einführen des Einlegeprofils 26 durch das Innenmaß des Versorgungsschlauches 17, 18 federnd zusammgedrückt werden, wobei nach Passieren des Schlauch-Endes 15, 16 ein federndes Zurückschnappen erfolgt.

Es ist eine erste Reihe 36a von in Profil-Längsrichtung hintereinander angeordneten Fixierzungen vorgesehen, die sich an der einen, mit der Verdickung 34 ausgestatteten Schmalseite des Basisabschnitts 33 befindet, und eine zweite Reihe 36b, die an der entgegengesetzten, ebenfalls mit einer Verdickung 34 versehenen Schmalseite des Basisabschnittes 33 vorgesehen ist. Dabei sind die Fixierzungen alternierend angeordnet, wobei auf Höhe der Lücke zwischen zwei hintereinander angeordneten Fixierzungen einer Reihe 36a eine Fixierzunge der anderen Reihe 36b ausgebildet ist und umgekehrt.

Am hinteren, also dem Einführabschnitt 31 gegenüberliegenden Ende des Einführprofils 26 befindet sich ein Handhabungsabschnitt 37, insbesondere in Form eines zylindrischen Zapfens, der eine Fixierung des Einlegeprofils 26 an einem geeigneten Einführwerkzeug (nicht dargestellt) ermöglicht.

Zur Montage werden zunächst die Versorgungsschläuche 17, 18 mit den zugeordneten Schlauchanschlussöffnungen 13, 14 des Temperierkissens 12 verbunden, insbesondere daran angeschweißt. Danach wird das Einführwerkzeug am runden Zapfen des Einlegeprofils 26 fixiert und das Einlegeprofil 26 mit dem Einführabschnitt 31 voraus in den zugeordneten Versorgungsschlauch 17, 18 eingeführt. Mit Hilfe des Einführwerkzeugs wird dann das Einlegeprofil 26 durch den Versorgungsschlauch 17, 18 hindurchgeschoben, wobei die biegeflexiblen Fixierzungen durch das Innenmaß des Schlauches zusammengedrückt werden. Das Einlegeprofil 26 wird solange durch den zugeordneten Versorgungsschlauch 17, 18 hindurchgeschoben, bis es am anderen Schlauch-Ende 15, 16 wieder austritt. Dabei schnappen die durch das Innenmaß des zugeordneten Versorgungsschlauches 17, 18 zunächst zusammengedrückten Fixierzungen wieder nach außen, so dass das Einlegeprofil 26 mit aufgeschnappter Fixierzunge einen größeren Querschnitt besitzt als das Innenmaß des Versorgungsschlauches 17, 18. Dadurch wird ein Zurückrutschen des Einlegeprofils 26 entgegen der Einführrichtung verhindert, da sich die zuletzt aus dem Versorgungsschlauch 17, 18 ausgetretene Fixierzunge an der Stirnseite des Schlauch-Endes 15, 16 abstützt. Die Fixierzungen bilden also Widerhaken, die ein Zurückrutschen des Einlegeprofils 26 verhindern. Gleichzeitig geben die Fixierzungen auch die axiale Fixierposition des Einlegeprofils 26 im zugeordneten Versorgungsschlauch 17, 18 vor. Ein weiteres Herausrutschen des Einlegeprofils in Einführrichtung aus dem Versorgungsschlauch 17, 18 heraus wird durch die mit Spannung an der Innenwandung des Versorgungsschlauches 17, 18 liegenden Fixierzungen gewährleistet, die noch nicht aus dem Schlauch-Ende 15, 16 herausgetreten sind.

Beim Einschieben kann sich der aus dem zugeordneten Schlauch-Ende 15, 16 herausgetretene Teil des Einlegeprofils 26 infolge seines biegeflexiblen Biegeabschnitts 32 an die Biegungen in der Kanalanordnung 20 des Temperierkissens 12 anpassen.

## Patentansprüche

1. Temperiereinrichtung zum Temperieren von Körperstellen des menschlichen Körpers, mit einem Temperierkissen (12), das mit einem Temperiergerät köppelbar, am Körper anlegbar und von temperiertem Fluid durchströmbar ist, mit wenigstens zwei Schlauchanschlussöffnungen (13, 14), in denen jeweils ein Schlauch-Ende (15, 16) eines Versorgungsschlauchs (17, 18) einer Schlauchanordnung (19) angeordnet oder einführbar ist, über die temperiertes Fluid im Kreislauf zwischen Temperiergerät und Temperierkissen (12) transportierbar ist, wobei temperiertes Fluid jeweils über einen Durchströmquerschnitt in das Temperierkissen (12) oder aus dem Temperierkissen (12) gelangt, wobei Aufhaltemittel (23, 25, 26) vorgesehen sind, die den Durchströmquerschnitt bei Abknicken des Temperierkissens (12) im Bereich der Schlauch-Enden (15, 16) zumindest teilweise offen halten, wobei die Aufhaltemittel wenigstens ein Einlegemittel aufweisen, das von der Stirnseite des Schlauch-Endes (15, 16) mit Überstand nach vorne abragt,
**dadurch gekennzeichnet, dass** das Einlegemittel ein im zugeordneten Versorgungsschlauch (17, 18) einlegbares, längliches Einlegprofil (26) ist, das im eingelegten Zustand von temperiertem Fluid umströmbar ist und dass Fixiermittel zur axialen Fixierung der gewünschten Einlegeposition des Einlegeprofils (26) vorgesehen sind.

2. Temperiereinrichtung nach Anspruch 1, **dadurch gekennzeichnet, dass** die Aufhaltemittel (23, 25, 26) derart ausgebildet sind, dass eine Biegeflexibilität des Temperierkissens (12) im Bereich der Schlauch-Enden (15, 16) erhalten bleibt.

3. Temperiereinrichtung nach Anspruch 1 oder 2, **dadurch gekennzeichnet, dass** die Aufhaltemittel (23, 25, 26) den Schlauch-Enden (15, 16) zugeordnet sind.

4. Temperiereinrichtung nach einem der vorhergehenden Ansprüche, **dadurch gekennzeichnet, dass** die Aufhaltemittel wenigstens einen von einer Stirnseite des Schlauch-Endes (15, 16) in Axialrichtung verlaufenden, in die Schlauchwandung eingebrachten Aufhalteausschnitt (23) aufweisen.

5. Temperiereinrichtung nach Anspruch 4, **dadurch gekennzeichnet, dass** zwei insbesondere diametral gegenüberliegende Aufhalteausschnitte (23) oder mehr als zwei Aufhalteausschnitte (23) vorgesehen sind.

6. Temperiereinrichtung nach einem der vorhergehenden Ansprüche, **dadurch gekennzeichnet, dass** die Aufhaltemittel einen von der Stirnseite des Schlauch-Endes (15, 16) nach vorne abragenden Aufhalteansatz (25) aufweisen.

7. Temperiereinrichtung nach Anspruch 1, **dadurch gekennzeichnet, dass** die Fixiermittel wenigstens ein, von einem Aussenumfang des Einlegeprofils (26) nach aussen abstehenden Fixiervorsprung (35) aufweisen, der sich zur Vorgabe einer axialen Fixierposition des Einlegeprofils (26) im Versorgungsschlauch (17, 18) an der Stirnseite des Schlauch-Endes (15, 16) widerhakenartig abstützt.

8. Temperiereinrichtung nach Anspruch 7, **dadurch gekennzeichnet, dass** der wenigstens eine Fixiervorsprung (35) von wenigstens einer biegeflexible Fixierzunge gebildet ist, die beim Einführen des Einlegeprofils (26) durch ein Innenmass des Versorgungsschlauches (17, 18) federnd zusammendrückbar ist und nach Passieren des Schlauch-Endes (15, 16) federnd zurückschnappt.

9. Temperiereinrichtung nach Anspruch 8, **dadurch gekennzeichnet, dass** mehrere Fixiervorsprünge (35) in Profil-Längsrichtung des Einlegeprofils (26) hintereinander angeordnet sind.

10. Temperiereinrichtung nach Anspruch 9, **dadurch gekennzeichnet, dass** wenigstens zwei Reihen mit jeweils hintereinander angeordneten Fixiervorsprüngen (35) vorgesehen sind, wobei vorzugsweise die Vorsprünge (35) einer ersten Reihe (36a) in Profil-Längsrichtung versetzt zu den Fixiervorsprüngen einer zweiten Reihe (36b) angeordnet sind.

11. Temperiereinrichtung nach einem der vorhergehenden Ansprüche, **dadurch gekennzeichnet, dass** die Schlauchanschlussöffnungen (13, 14) Bestandteil eines im Temperierkissen (12) angeordneten Schlauchanschlussstücks (27) sind, an dem sich die Aufhaltemittel (25) befinden oder dem die Aufhaltemittel (25) zugeordnet sind.

12. Temperiereinrichtung nach einem der Ansprüche 1 bis 11,
**dadurch gekennzeichnet, dass** das Einlegeprofil (26) einen Einführabschnitt (31) mit gegenüber dem Rest des Einlegeprofils (26) grösseren Querschnitt zum Einführen in die zugeordnete Schlauchanschlussöffnung (13, 14) des Temperierkissens (12) aufweist.

13. Temperiereinrichtung nach einem der Ansprüche 1 bis 12, **dadurch gekennzeichnet, dass** das Einlegeprofil (26) einen Insbesondere in Profil-Längsrichtung an den Einführabschnitt (31) anschliessenden, biegeflexiblen Biegeabschnitt (33) aufweist, mit dem eine Führung des Einlegeprofils (26) in den Biegungen der Kanalordnung (20) des Temperierkissens (12) möglich ist.

## Claims

1. Temperature control device for controlling the temperature of parts of the human body, with a temperature control cushion (12), which can be coupled to a temperature control appliance and can be placed on the body and through which temperature-controlled fluid can flow, with at least two tube connection openings (13, 14) in which a respective tube end (15, 16) of a supply tube (17, 18) of a tube arrangement (19) is arranged or can be inserted, through which tube arrangement (19) temperature-controlled fluid can be transported in the circuit between temperature control appliance and temperature control cushion (12), wherein temperature-controlled fluid flows into the temperature control cushion (12) or out of the temperature control cushion (12) in each case via a cross-sectional area of flow, wherein holding means (23, 25, 26) are provided that keep the cross-sectional area of flow at least partially open in the event of buckling of the temperature control cushion (12) in the area of the tube ends (15, 16), wherein the holding means have at least one insert means that protrudes forwards from the front face of the tube end (15, 16), **characterized in that** the insert means is an elongate insert profile (26) which can be inserted in the assigned supply tube (17, 18) and around which temperature-controlled fluid can flow in the inserted state, and **in that** fixing means are provided for axially fixing the desired insertion position of the insert profile (26).

2. Temperature control device according to Claim 1, **characterized in that** the holding means (23, 25, 26) are designed in such a way that a bending flexibility of the temperature control cushion (12) in the area of the tube ends (15, 16) is maintained.

3. Temperature control device according to Claim 1 or 2, **characterized in that** the holding means (23, 25, 26) are assigned to the tube ends (15, 16).

4. Temperature control device according to one of the preceding claims, **characterized in that** the holding means have at least one holding cutout (23) formed in the tube wall and extending axially from a front face of the tube end (15, 16).

5. Temperature control device according to Claim 4, **characterized in that** two in particular diametrically opposite holding cutouts (23) are provided, or more than two holding cutouts (23) are provided.

6. Temperature control device according to one of the preceding claims, **characterized in that** the holding means have a holding extension (25) protruding forwards from the front face of the tube end (15, 16).

7. Temperature control device according to Claim 1, **characterized in that** the fixing means have at least one fixing projection (35) which protrudes outwards from an outer circumference of the insert profile (26) and which, in order to define an axial fixing position of the insert profile (26) in the supply tube (17, 18), supports itself like a barb on the front face of the tube end (15, 16).

8. Temperature control device according to Claim 7, **characterized in that** the at least one fixing projection (35) is formed by at least one flexible fixing tongue which, upon insertion of the insert profile (26), can be compressed together resiliently by an inside dimension of the supply tube (17, 18) and snaps back resiliently after passing the tube end (15, 16).

9. Temperature control device according to Claim 8, **characterized in that** several fixing projections (35) are arranged one behind another in the longitudinal direction of the insert profile (26).

10. Temperature control device according to Claim 9, **characterized in that** at least two rows of fixing projections (35) arranged one behind another are provided, wherein preferably the projections (35) of a first row (36a) are offset with respect to the fixing projections of a second row (36b) in the longitudinal direction of the profile.

11. Temperature control device according to one of the preceding claims, **characterized in that** the tube connection openings (13, 14) are part of a tube connection piece (27), which is arranged in the temperature control cushion (12) and on which the holding means (25) are located or to which the holding means (25) are assigned.

12. Temperature control device according to one of Claims 1 to 11, **characterized in that** the insert profile (26) has an insertion portion (31), which has a greater cross section than the rest of the insert profile (26) and which is inserted into the assigned tube connection opening (13, 14) of the temperature control cushion (12).

13. Temperature control device according to one of Claims 1 to 12, **characterized in that** the insert profile (26) has a flexible bending portion (33), which adjoins the insertion portion (31) particularly in the longitudinal direction of the profile and with which the insert profile (26) can be guided into the bends of the channel arrangement (20) of the temperature control cushion (12).

## Revendications

1. Dispositif de régulation de la température servant à réguler la température de zones corporelles du corps humain, avec un coussin de régulation de la température (12), qui peut être couplé à un appareil de régulation de la température, appliqué sur le corps et parcouru par un fluide à température régulée, avec au moins deux ouvertures de raccordement de tuyau (13, 14), dans chacune desquelles une extrémité de tuyau (15, 16) d'un tuyau d'alimentation (17, 18) d'un agencement de tuyaux (19) est disposée ou peut être introduite, par lequel un fluide à température régulée peut être transporté en circuit entre l'appareil de régulation de la température et le coussin de régulation de la température (12), un fluide à température régulée circulant chaque fois à travers une section transversale d'écoulement dans le coussin de régulation de la température (12) et hors du coussin de régulation de la température (12), des moyens d'ouverture (23, 25, 26), qui maintiennent la section transversale d'écoulement au moins partiellement ouverte lors du pliage du coussin de régulation de la température (12) dans la région des extrémités de tuyau (15, 16), étant prévus, les moyens d'ouverture présentant au moins un moyen d'insertion, qui est saillant avec un dépassement vers l'avant à partir de la face frontale de l'extrémité de tuyau (15, 16), **caractérisé en ce que** le moyen d'insertion est un profilé d'insertion allongé (26), insérable dans le tuyau d'alimentation associé (17, 18), qui à l'état inséré peut être contourné par le fluide à température régulée, et **en ce qu'**il est prévu des moyens de fixation pour la fixation axiale de la position d'insertion souhaitée du profilé d'insertion (26).

2. Dispositif de régulation de la température selon la revendication 1, **caractérisé en ce que** les moyens d'ouverture (23, 25, 26) sont réalisés de telle manière qu'il subsiste une souplesse de flexion du coussin de régulation de la température (12) dans la région des extrémités de tuyau (15, 16).

3. Dispositif de régulation de la température selon la revendication 1 ou 2, **caractérisé en ce que** les moyens d'ouverture (23, 25, 26) sont associés aux extrémités de tuyau (15, 16).

4. Dispositif de régulation de la température selon l'une quelconque des revendications précédentes, **caractérisé en ce que** les moyens d'ouverture présentent au moins une section d'ouverture (23) introduite dans la paroi du tuyau et s'étendant en direction axiale à partir d'une face frontale de l'extrémité de tuyau (15, 16).

5. Dispositif de régulation de la température selon la revendication 4, **caractérisé en ce qu'**il est prévu deux sections d'ouverture (23), en particulier diamétralement opposées, ou plus de deux sections d'ouverture (23).

6. Dispositif de régulation de la température selon l'une quelconque des revendications précédentes, **caractérisé en ce que** les moyens d'ouverture présentent un prolongement d'ouverture (25) en saillie vers l'avant à partir de la face frontale de l'extrémité de tuyau (15, 16).

7. Dispositif de régulation de la température selon la revendication 1, **caractérisé en ce que** les moyens de fixation présentent au moins une protubérance de fixation (35) saillante vers l'extérieur sur un contour extérieur du profilé d'insertion (26), qui s'appuie à la manière d'un rétro-crochet sur la face frontale de l'extrémité de tuyau (15, 16) pour préciser une position de fixation axiale du profilé d'insertion (26) dans le tuyau d'alimentation (17, 18).

8. Dispositif de régulation de la température selon la revendication 7, **caractérisé en ce que** ladite au moins une protubérance de fixation (35) est formée par au moins une languette de fixation souple en flexion, qui est élastiquement compressible lors de l'introduction du profilé d'insertion (26) à travers une dimension intérieure du tuyau d'alimentation (17, 18) et s'écarte de nouveau élastiquement après avoir franchi l'extrémité de tuyau (15, 16).

9. Dispositif de régulation de la température selon la revendication 8, **caractérisé en ce que** plusieurs protubérances de fixation (35) sont disposées l'une derrière l'autre dans la direction longitudinale de profilé du profilé d'insertion (26).

10. Dispositif de régulation de la température selon la revendication 9, **caractérisé en ce qu'**il est prévu au moins deux rangées de protubérances de fixation (35) disposées respectivement l'une derrière l'autre, les protubérances (35) d'une première rangée (36a) étant de préférence décalées, dans la direction longitudinale de profilé, par rapport aux protubérances de fixation d'une seconde rangée (36b).

11. Dispositif de régulation de la température selon l'une quelconque des revendications précédentes, **caractérisé en ce que** les ouvertures de raccordement de tuyau (13, 14) font partie d'une pièce de raccordement de tuyau (27) disposée dans le coussin de régulation de la température (12), sur laquelle les moyens d'ouverture (25) sont situés ou à laquelle les moyens d'ouverture (25) sont associés.

12. Dispositif de régulation de la température selon l'une quelconque des revendications 1 à 11, **caractérisé en ce que** le profilé d'insertion (26) comprend une section d'introduction (31) présentant, par rapport au reste du profilé d'insertion (26), une plus grande section transversale destinée à l'introduction dans l'ouverture de raccordement de tuyau associée (13, 14) du coussin de régulation de la température (12).

13. Dispositif de régulation de la température selon l'une quelconque des revendications 1 à 12, **caractérisé en ce que** le profilé d'insertion (26) présente une section de flexion souple en flexion (33), se raccordant à la section d'introduction (31) en particulier dans la direction longitudinale du profilé, par laquelle un guidage du profilé d'insertion (26) dans les courbes du réseau de canaux (20) du coussin de régulation de la température (12) est possible.
